(19) Europäisches Patentamt
European Patent Office
Office européen des brevets

(11) EP 0 660 825 B1

# (12) EUROPÄISCHE PATENTSCHRIFT

(45) Veröffentlichungstag und Bekanntmachung des Hinweises auf die Patenterteilung:
**03.12.1997 Patentblatt 1997/49**

(21) Anmeldenummer: **93919327.2**

(22) Anmeldetag: **13.09.1993**

(51) Int. Cl.$^6$: **C07D 225/02**, C12P 17/10, C07F 7/18, A61K 31/395, A61K 31/695 // (C12P17/10, C12R1:465)

(86) Internationale Anmeldenummer:
**PCT/EP93/02472**

(87) Internationale Veröffentlichungsnummer:
**WO 94/06774 (31.03.1994 Gazette 1994/08)**

## (54) ENTZÜNDUNGSHEMMENDE MAKROLACTAME (CYCLAMENOL)

ANTI-INFLAMMATORY MACROLACTAM (CYCLAMENOL)

MACROLACTAME (CYCLAMENOL) ANTI-INFLAMMATOIRE

(84) Benannte Vertragsstaaten:
**AT BE CH DE DK ES FR GB GR IE IT LI LU MC NL PT SE**

(30) Priorität: **18.09.1992 DE 4231289**

(43) Veröffentlichungstag der Anmeldung:
**05.07.1995 Patentblatt 1995/27**

(73) Patentinhaber: **BAYER AG**
**51368 Leverkusen (DE)**

(72) Erfinder:
- **MÜLLER, Hartwig**
  **D-42553 Velbert (DE)**
- **BISCHOFF, Erwin**
  **D-42115 Wuppertal (DE)**
- **FIEDLER, Volker-Bernd**
  **D-51382 Leverkusen (DE)**
- **WEBER, Karlheinz**
  **D-42115 Wuppertal (DE)**
- **FUGMANN, Burkhard**
  **D-40878 Ratingen (DE)**
- **ROSEN, Bruno**
  **D-42489 Wülfrath (DE)**

(56) Entgegenhaltungen:
- **THE JOURNAL OF ANTIBIOTICS Bd. 37, Nr. 1 , Januar 1984 , TOKYO JP Seiten 13 - 19 N. ONISHI ET AL. 'A Macrocyclic Antibiotic M-230B produced by Myxococcus Xanthus. Isolation and Characterization.' in der Anmeldung erwähnt**

EP 0 660 825 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.15.2/3.4

## Beschreibung

Die Erfindung betrifft den neuen Naturstoff Cyclamenol, ein Verfahren zu seiner Herstellung aus Mikroorganismen aus der Ordnung der Actinomycetales, insbesondere aus dem Streptomyces-Stamm MHW 846, chemische Derivate, sowie ihre Verwendung als Arzneimittel, insbesondere für akute und chronisch entzündliche Erkrankungen in der Human- und Tiermedizin.

Es ist bereits bekannt, daß eine Reihe mikrobiell gewonnener makrocyclischer Lactame, wie beispielsweise Hitachimycin, Virginiamycin oder Myxovirescin eine antibakterielle oder Antitumorwirkung besitzen [vgl. z.B. Pure Appl. Chem. 61, 405-408 (1989); 13 (1984);]. Antibiotics 35, 1454-1459 (1982)].

Die vorliegende Erfindung betrifft jetzt Verbindungen der allgemeinen Formel (I)

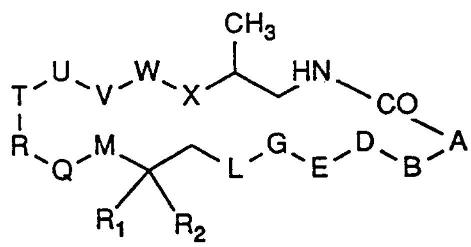

(I)

in welcher

A, B, D, E, G, L, M, Q, R, T, U, V, W und X jeweils für die >$CH_2$-Gruppe stehen und

$R^1$ und $R^2$ für Wasserstoff stehen oder

$R^1$ für Wasserstoff steht und

$R^2$ für Hydroxy steht, oder

$R^1$ und $R^2$ gemeinsam die =O-Gruppe bilden, oder

A und B, D und E, G und L, M und Q, R und T, U und V und W und X jeweils für die -CH=CH-Gruppe stehen,

$R^1$ für Wasserstoff steht und

$R^2$ für Hydroxy steht (Cyclamenol) oder für die Gruppe der Formel $-O-CO-CH_3$ oder $-O-Si(CH_3)_3$ steht.

Überraschenderweise zeichnen sich sowohl der neue Naturstoff Cyclamenol als auch seine chemischen Derivate durch eine starke inhibierende Wirkung auf zentrale Vorgänge bei akuten und chronischen entzündlichen Prozessen aus.

Außerdem wurden Verfahren zur Herstellung der erfindungsgemäßen Verbindungen der allgemeinen Formel (I) gefunden, wobei zunächst die Gewinnung des Naturstoffs Cyclamenol der Formel (Ia)

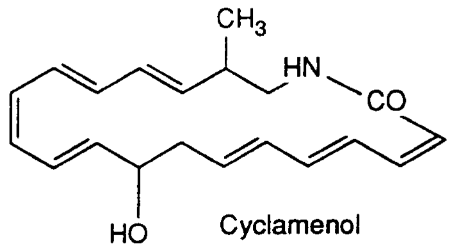

(Ia)

Cyclamenol

beschrieben wird.

Die Verbindung der allgemeinen Formel (Ia) wird gewonnen, indem man den Stamm Streptomyces spec. MHW 846 in einem Nährmedium züchtet und anschließend daraus isoliert.

Die erfindungsgemäße Verbindung der allgemeinen Formel (Ia) wird durch die Kultur eines Streptomyceten-Stammes in geeigneten Nährlösungen unter geeigneten physiologischen Bedingungen erzeugt. Sie wird aus der Kulturlösung durch Extraktion oder durch Absorption abgetrennt und durch weitere geeignete Methoden angereichert.

Desweiteren betrifft die vorliegende Erfindung Mikroorganismen der Familie Streptomycetaceae, die bei der Kultivierung in einem Kohlenstoff- und Stickstoffquellen sowie Mineralsalze enthaltenden Nährmedium eine Verbindung produzieren, welche die bei der Substanzcharakterisierung im Folgenden unter (1) bis (6) aufgeführten Eigenschaften und Parameter aufweist.

Für das Herstellungsverfahren ist der Stamm Streptomyces spec. MHW 846 aus der Ordnung der Actinomycetales, Familie Streptomycetaceae, Gattung Streptomyces oder von ihm abstammende Varianten und Mutanten besonders wichtig. Der Stamm wurde von einer Bodenprobe aus Wahuya, Miyagi in Japan isoliert. Eine Kultur dieses Stammes wurde bei der Deutschen Sammlung von Miliroorganismen in Braunschweig am 14.12.88 unter der Nummer DSM 4950 hinterlegt.

Aufgrund der chemotaxonomischen Untersuchungen auf DL-DAP nach C.S. Cummins und H. Harris, J. Gen. Microbiol. 15, 9-10, 1956 und auf iso/anteiso Fettsäuremuster nach R.M. Kroppenstedt in Chemical Methods in Bacterial Systematics (eds. Goodfellow, M.; Minnikin, O.E.) 173 - 199, 1985 wurde der Stamm der Gattung Streptomyces zugeordnet. Es handelt sich um einen Streptomyceten, der auf den Medien nach E.B. Shirling and D. Gottlieb, Int. Journal Syst. Bact. 16, 313-340, 1966, nur spärlich Luftmycel bildet, so daß die Luftmycelfarbe nicht eindeutig feststellbar ist.

Streptomyces spec. MHW 846 bildet auf Pepton-Eisen-Agar und Trypsin-Agar melanoides Pigment. Von den angebotenen C-Quellen wird nur D-Glucose eindeutig verwertet (Tab. 1).

Der Stamm wächst in einem Temperaturbereich von +20°C bis +35°C. Bei +5°C und +45°C ist kein Wachstum zu beobachten.

Tabelle 1

| C-Quellen-Verwertung von Stamm Streptomyces spec. MHW 846 DSM 4950. | |
|---|---|
| Verwertung von | Ergebnis: |
| L-Arabinose | - |
| Saccharose | - |
| D-Xylose | - |
| Inositol | - |
| D-Mannit | - |
| D-Fructose | - |
| Rhamnose | - |
| Raffinose | - |
| Cellulose | - |
| D-Glucose | + |
| + = Verwertung<br>- = keine Verwertung | |

Die Anreicherung und Isolierung des Stammes erfolgte nach den üblichen Methoden der Actinomyceten-Isolierung durch Ausplattieren von Erdprobensuspensionen auf Petrischalen, vier- bis sechswöchtige Bebrütung und wiederholte Abimpfung von Einzelkolonien [Williams, S.T. and Cross, T. (1971), Actinomycetes, in: Methods in Microbiology 4, 295 - 334, Booth, C. (editor), London - New York: Academic Press].

Es wurde weiterhin gefunden, daß man die erfindungsgemäße Verbindung (Ia) erhält, wenn man geeignete Mikroorganismen der Familie Streptomycetaceae in einem Nährmedium, welches assimilierbare Kohlenstoff- und Stickstoffquellen sowie Mineralsalze enthält, unter aeroben Bedingungen kultiviert und die Verbindung nach üblichen Methoden isoliert.

Für das Verfahren zur Herstellung der erfindungsgemäßen Verbindung (Ia) verwendet man Nährmedien, die die üblichen Kohlenstoff- und Stickstoffquellen und die notwendigen Salze enthalten. Als Kohlenstoffquelle können verwendet werden:

Kohlenhydrate, insbesondere Polysaccharide, wie z.B. Stärke, Monosaccharide, wie z.B. Glucose. Weiterhin können natürlich vorkommende Gemische, wie z.B. Malzextrakt, sowie Gemische aus den erwähnten Komponenten verwendet werden.

Als N-Quelle können die üblichen stickstoffhaltigen Nährmedienbestandteile Verwendung finden, so z.B. Aminosäuren, Eiweißstoffe, Eiweißhydrolysate, Ammoniumsalze, natürlich vorkommende komplexe Stoffe, wie Sojabohnenmehl, Milchpulver und geeignete Mischungen derselben.

Als Hilfsstoffe werden im Nährmedium Mineralsalze benötigt, z.B. Phosphate, Sulfate oder Chloride des Kaliums, des Natriums, des Calciums, des Magnesiums, Eisens, Zinks und Mangans. Die Konzentration dieser Stoffe kann in weiten Grenzen schwanken, z.T. sind die notwendigen Konzentrationen der Mineralsalze in den o.a. Kohlenstoff- oder Stickstoffquellen oder im verwendeten Wasser als Verunreinigungen enthalten.

Weiterhin können als Hilfsstoffe noch Antischaummittel der verschiedensten Art Verwendung finden, wie z.B. Polyole oder Silikone.

Das Verfahren zur Herstellung der erfindungsgemäßen Verbindung (Ia) kann mit Hilfe üblicher fester, halbfester oder flüssiger Nährmedien durchgeführt werden. Bevorzugt werden wäßrig flüssige Nährmedien.

Die Beimpfung der Nährmedien erfolgt dabei nach allgemein üblichen Methoden, z.B. über Schrägröhrchen oder Kolbenkulturen.

Die Kultur erfolgt unter aeroben Bedingungen und kann gemäß den allgemein üblichen Methoden wie unter Verwendung von Schüttelkulturen, z.B. Schüttelkolben, von luftbewegten Kulturen oder von Submerskulturen durchgeführt werden. Bevorzugt erfolgt die Kultivierung in aeroben Submersverfahren in belüfteten Fermentern, z.B. in üblichen Submersfermentationstanks. Es ist möglich, die Kultur kontinuierlich oder diskontinuierlich durchzuführen. Vorzugsweise wird diskontinuierlich gearbeitet.

Bei der Durchführung des Herstellungsverfahrens wird unter aeroben Bedingungen gearbeitet: die Kultur kann gemäß üblicher Methoden, so z.B. unter Verwendung von Schüttelkulturen oder von belüfteten Fermenterkulturen, durchgeführt werden. Die Prozentverhältnisse der Nährlösungsbestandteile können in weiten Bereichen schwanken, im allgemeinen machen die Kohlenstoffquellen 0,5 bis 8%, vorzugsweise 0,6 bis 6%, die Stickstoffquellen 0,1 bis 5%, vorzugsweise 0,5 bis 2% aus, die Salze liegen in üblichen Konzentrationen vor, vorzugsweise im Bereich zwischen 0,001 bis 0,5 Gew.-%. Die Antischaummittel liegen in bis zu 0,5%iger Konzentration vor. Die zur Sterilisation angewandten Temperaturen liegen bei +100 bis +140°C, bevorzugt bei +120 bis +130°C:

Der pH-Wert der wachsenden Kultur sollte vorzugsweise zwischen etwa 6 bis etwa 8,5, insbesondere zwischen 6,5 und 8,0 gehalten werden. Ein zu starker pH-Abfall in den sauren Bereich kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säuren, zB. $H_2SO_4$, oder sterile Laugen, z.B. NaOH in Abständen in die Kulturlösung eingespritzt werden.

Es ist zweckmäßig sicherzustellen, daß die Mikroorganismen ausreichend mit Sauerstoff sowie Nährstoffen in Kontakt gebracht werden. Dies kann nach den allgemein üblichen Methoden wie Schütteln und Rühren erfolgen.

Die Züchtungstemperatur kann zwischen etwa +16°C und +42°C, vorzugsweise zwischen +24°C und +32°C liegen, besonders bevorzugt liegt sie bei etwa +28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiellen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 7, vorzugsweise 1 bis 4 Tage nach Züchtungsbeginn erreicht.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtung können z.B. Dampf- oder Trockensterilisation verwendet werden.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxyethlyen- bzw. Polyoxypropylenverbindungen zugesetzt werden. Schaum kann auch mit Hilfe der üblichen mechanischen Vorrichtungen (welche z.B. Zentrifugalkräfte benutzen) gedämpft oder beseitigt werden.

Die neue Verbindung (Ia) ist sowohl im Kulturüberstand als auch im Mycel zu finden und kann mit Hilfe von üblichen Extraktions- und/oder Chromatographieverfahren aus dem Fermentationsansatz isoliert und gegebenenfalls gereinigt werden. Die Chromatographie kann in Form der Säulenchromatographie durchgeführt werden. Mit Erfolg läßt sich auch die Hochdruckflüssigkeitschromatographie (HPLC) einsetzen. Als Absorptionsmittel können die üblichen anorganischen oder organischen Adsorptionsmittel eingesetzt werden, wie z.B. Kieselgel, Magnesiumsilikat, Aktivkohle, Cellulose, Cellulosederivate, Kunstharze wie Polyamide, z.B. acetyliertes Polyamid, Dextrangele oder modifizierte Dextrangele. Als Laufmittel können die verschiedensten Lösemittel oder Lösemittelgemische verwendet werden, in welchen die erfindungsgemäße Verbindung löslich ist Bevorzugt werden Essigsäureethylester, Chloroform und Methanol oder ihre Gemische (beispielsweise Gemische aus Chloroform und Methanol oder aus Essigsäureethylester und Chloroform) eingesetzt.

Bevorzugt werden zur Isolierung der erfindungsgemäßen Verbindung (Ia) Chromatographie-Verfahren verwendet, z.B. unspezifische Adsorption an Sorbentien wie Kieselgel oder Geldiffusionschromatographie. Dies sind die von der Reinigung schlecht wasserlöslicher Naturstoffe her bekannten Methoden.

Aus ihren Lösungen kann die erfindungsgemäße Verbindung (Ia) nach den üblichen Methoden, z.B. Verdampfen des Lösemittels, Gefriertrocknung usw. erhalten werden.

In einer bevorzugten Ausführungsform wird das Mycelium aus der Kulturbrühe, vorzugsweise durch Zentrifugation abgetrennt und mit einem weniger polaren Lösemittel mehrfach, vorzugsweise zweimal extrahiert. Als Lösemittel können $C_1$-$C_4$-Alkohole, $C_1$-$C_4$-Ketone oder halogenierte Kohlenwasserstoffe verwendet werden. Die wäßrig-organische Lösung wird im Vakuum, z.B. etwa auf 1/20 des Volumens der Kulturbrühe konzentriert und weiter verarbeitet. Die Kulturbrühe wird nach Abtrennen des Mycels mehrfach mit einem nicht mit Wasser mischbaren Lösemittel, bevorzugt Essigsäureethylester extrahiert und nach Konzentration auf etwa 1/10 des Ausgangsvolumens weiter verarbeitet.

Aus diesen beiden Rohextrakten kann die erfindungsgemäße Verbindung (Ia) durch übliche chromatographische Methoden, vorzugsweise Chromatographie an Kieselgel isoliert werden.

Sie kann außerdem an unspezifischen Adsorberharzen auf Polystyrolbasis (z.B. Amberlite XAD der Firma Roehm und Haas oder Lewapol CA 9221 der Firma Bayer) gebunden werden. Die Desorption wird fraktioniert, durch Mischungen aus Wasser und den oben angegebenen organischen Lösemitteln, insbesondere Wasser / Methanol vorgenommen.
Die durch Test ermittelten aktiven Fraktionen werden unter reduziertem Druck bis zur vollständigen Entfernung des organischen Lösemittels bei +30°C bis +35°C konzentriert und gefriergetrocknet.

Das Lyophilisat wird erneut in Wasser suspendiert und vorzugsweise mit Essigsäureethylester oder anderen nicht mit Wasser mischbaren Lösemitteln extrahiert. Aus dem Extrakt wird die erfindungsgemäße Verbindung durch übliche

chromatographische Methoden, vorzugsweise Chromatographie an Kieselgel, gewonnen.

Die erfindungsgemäße Verbindung (Ia) wird durch folgende Eigenschaften charakterisiert:

1) Der Naturstoff ist ein farbloser bis blaßgelber Feststoff. Er sintert beim Erwärmen und zersetzt sich > 150°C. Der optische Drehwert beträgt sofort nach dem Lösen $[\alpha]^{20}_{589}$ = + 3840° (c = 0,5% in DMF). $R_f$ = 0,45 (Fließmittelsystem Chloroform : Methanol (9:1), Fertigplatten Kieselgel 60, $F_{254}$ (Merck), grüne Farbe mit TDM-Reagens, graubraune Farbe mit Jod).

2) UV Spektrum (Abb. 1, s. Anlage) Die Aufnahme erfolgt in Methanol (c = 0,1 mg in 1 ml $CH_3OH$). Saurer bzw. alkalischer pH werden durch Zusatz von 20 µl 1 N HCl bzw. 1 N Natronlauge pro ml Lösung eingestellt.

Tabelle 2

| Maxima [λ max.] und Extinktionen [$E^{1\%}_{cm}$] der Verbindung (Ia) | | |
|---|---|---|
| | λmax. [µm] | [$E^{1\%}_{cm}$] |
| neutral | 213,5 | 416 |
| | 276,0 | 2642 |
| | 285,0 | 3228 |
| sauer | 228 | 294 |
| | 275,5 | 2262 |
| | 285,5 | 2812 |
| alkalisch | 215,5 | 368 |
| | 276,0 | 2300 |
| | 285,5 | 2834 |

3) Das IR-Absorptionsspektrum der Verbindung (Ia) wird in Abb. 2 wiedergegeben (Abszisse: Wellenzahl in $cm^{-1}$; Ordinate: Absorption). Wird die Substanz in KBr-Preßlingen verpreßt, so zeigt sie bei folgenden Wellenzahlen (ausgedrückt in $cm^{-1}$) Absorptionsbanden:

<u>Relative Intensitäten:</u>

vs = sehr stark
s = stark
m = mittel
w = schwach
sh = Schulter

Tabelle 3

| Wellenzahl in $cm^{-1}$ | Wellenzahl in cm-[1] |
|---|---|
| 3400 (sh) | 1365 (m) |
| 3300 (vs) | 1330 (w) |
| 3250 (sh) | 1280 (w) |
| 3020 (w) | 1220 (w) |
| 2945 (w) | 1120 (w) |
| 2855 (w) | 1030 (s) |
| 1655 (s) | 1000 (vs) |
| 1610 (m) | 875 (w) |
| 1600 (w) | 850 (w) |
| 1550 (sh) | 800 (w) |
| 1530 (m) | 725 (w) |
| 1445 (m) | |

4) Das $^{1}H$-Kernresonanzspektrum gibt die Signallage in Teilen pro Million (ppm) und Schwingungen pro Sekunde gemäß Abb. 3 an. Es wurde in einer Dimethylsulfoxid-$d_6$-Lösung der Verbindung mit TMS als Standard (intern) mit einem Kernresonanz-Spektrometer der Firma Bruker bei einer Feldstärke von 250 MHz aufgenommen.
Durch HIC-Heteroatom-Korrelationsspektroskopie konnten den Kohlenstoffatomen die an sie gebundenen Wasserstoffatome zugeordnet werden:

Tabelle 4

| chem. Verschiebungen der C-Atome (ppm) | Zuordnung | H-Korrelation (ppm) |
|---|---|---|
| 166,3 | CO-N< | - |
| 139,0 | CH= | 6,80 |
| 137,8 | CH= | 6,35 |
| 136,1 | CH= | 5,10 |
| 135,9 | CH= | 5,70 |
| 135,1 | CH= | 5,20 |
| 132,8 | CH= | 5,45 |
| 132,7 | CH= | 5,95 |
| 132,4 | CH= | 5,90 |
| 132,3 | CH= | 5,63 |
| 131,7 | CH= | 5,80 |
| 131,0 | CH= | 6,05 |
| 130,8 | CH= | 5,98 |
| 129,1 | CH= | 6,00 |
| 124,3 | CH= | 5,75 |
| 72,8 | CH<O | 4,1 |
| 44,3 | $CH_2$ | 3,4 / 2,85 |
| 42,0 | $CH_2$ | 2,65 / 1,95 |
| 40,7 | CH | 2,2 |
| 17,1 | $CH_3$ | 1,0 |
| Durch H/D-Austausch wurden eine OH-Gruppe bei $\delta$ = 5,0 ppm und eine NH-Gruppe bei $\delta$ = 7,5 ppm identifiziert. | | |

Die Verknüpfung der C-Atome miteinander und somit die Grundstruktur des Moleküls kann durch Bestimmung der Kopplung der entsprechenden an sie gebundenen Wasserstoff-Atome im zweidimensionalen $^1H$, $^1H$-COSY-NMR-Spektrum festgestellt werden. Hierzu wird die chemische Verschiebung jedes H-Atoms im 2D-Plot bestimmt (s. Tabelle 4).
Die Kopplungspartner werden im Korrelationsdiagramm festgelegt. Als Beispiel werden in Abb. 3 die Kopplungspartner des H-Atoms bei $\delta$ = 4,1 ppm identifiziert: eine OH-Gruppe bei 5,0 ppm, ein olefinisches H bei 5,2 ppm sowie zwei diastereotope Protonen bei $\delta$ = 1,95 und 2,65 ppm.
Zwischen 5,1 und 6,1 ppm liegen 12 teilweise kompliziert miteinander koppelnde Protonen. Alle Signale lassen sich in eine Korrelationsgraphik einordnen (Tabelle 5):

Tabelle 5

| δ des H-Atoms | Gruppe | Signalmultiplizität |
|---|---|---|
| 1,0 | $CH_3$ | d(d) |
| 1,95 | CH | dd |
| 2,2 | CH | sept(t) |
| 2,65 | CH | m |
| 2,85 | CH | dd |
| 3,40 | CH | dd |
| 4,1 | CH<O | ddd |
| 5,0 | OH | d |
| 7,5 | NH | dd |
| 5,1 | CH= | dd |
| 5,2 | CH= | dd |
| 5,45 | CH= | ddd |
| 5,63 | CH= | dd |
| 5,70 | CH= | m(dd) |
| 5,75 | CH= | m |
| 5,80 | CH= | dd |
| 5,85 - 6,10 | CH= 5x) | mm |
| 6,35 | CH= | dd |
| 6,80 | CH= | dd |

Mit Hilfe eines 600 MHz-Spektrums (Bruker, Karlsruhe) konnten alle Verschiebungen zugeordnet werden. Demnach weisen 3 Doppelbindungen mit $J^3$-Kopplungskonstanten der Protonen zwischen 14,5 Hz und 15,5 Hz trans- und eine mit 11,5 Hz cis-Konfiguration auf.

6) Die Verbindung (Ia) zeigt bei der Massenspektroskopie unter DCI-Bedingungen einen Molpeak bei m/z = 311 . Das Massenspektrum im EI-Modus ergibt für den Molpeak eine Hochauflösung von 311, 1895 (berechnet für $C_{20}H_{25}NO_2$ = 311, 1885).
Die übrigen erfindungsgemäßen Verbindungen, die unter der allgemeinen Formel (I) erfaßt sind, können durch chemische Derivatisierung des Naturstoffs Cyclamenol der allgemeinen Formel (Ia) hergestellt werden, wobei diese dann wiederum einen Beweis für dessen Konstitution darstellen.

Dabei wird der Naturstoff der Formel (Ia)

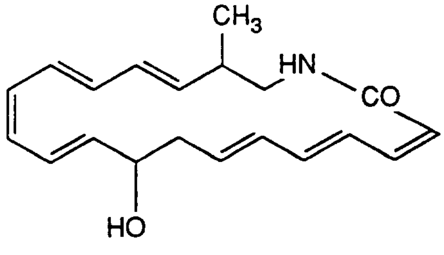

(Ia)

[A] im Fall, daß $R^2$ (allgemeine Formel I) für Acetyloxy steht, unter Schutzgasatmosphäre in Pyridin mit Acetanhydrid in Anwesenheit von 4-(N,N-Dimethylamino)pyridin bei Raumtemperatur umgesetzt,

[B] im Fall, daß $R^2$ (allgemeinen Formel I) für die Trimethylsilyloxygruppe steht, in Acetonitril, ebenfalls unter Schutzgasatmosphäre in Anwesenheit von Pyridin und Trimethylchlorsilan mit N,O-Bis(trimethylsilyl)acetamid bei Raumtemperatur umgesetzt,
und

[C] im Fall ($R^1$ = H / $R^2$ = H), ($R^1$ = H, $R^2$ = OH), ($R^1$ + $R^2$ = =O) und A-X jeweils für die -$CH_2$-Gruppe stehen (allgemeine Formel I), in Methanol, in Anwesenheit von Palladium auf Aktivkohle mit Wasserstoff bei 0°C hydriert.

Die erfindungsgemäßen Verbindungen der allgemeinen Formel (I) und (Ia) können als Wirkstoffe in Arzneimitteln bei akuten und chronischen entzündlichen Prozessen und anderen pathologischen Zuständen, an denen weiße Blutzellen beteiligt sind, eingesetzt werden. Ihre Wirkungen bestehen in der starken Inhibition der Freisetzung von Sauerstoffradikalen, hydrolytisch wirksamen Enzymen wie beispielsweise β-Glucuronidase und Elastase und der oxidativ wirkenden Myeloperoxidase aus humanen polymorphkernigen Neutrophilen.

Sie sind somit bevorzugt zur Behandlung und Verhütung von akuten und chronischen Entzündungen der Atemwege, wie Allergien / Asthma, Bronchitis, Rheuma, Arteriosklerose, Arthrose, Entzündungen des Gastrointestinaltraktes, Myokarditis und Herzinfarkt geeignet.

Anwendungsbeispiel

1. Isolierung der Myeloperoxidase:
Für die Messungen wurde Myeloperoxidase aus humanen polymorphkernigen Neutrophilen verwendet Diese wurden aus frischem venösen Vollblut nach der Methode von D. English und B.R. Andersen, Journal of Immunological Methods 5, (1974) 249 - 252 isoliert.
$10^7$ Zellen wurden mit 1 ml 0,5% Ceryltrimethylammoniumbromid in Na-Phosphatpuffer 50 mmol/l pH 6,0 10 min bei 0°C lysiert, eingefroren und direkt wieder aufgetaut. Dieser Vorgang wurde zweimal wiederholt. Nun wurde 10 min bei 40.000 g zentrifugiert. Der Überstand wurde entsprechend seiner Aktivität an Myeloperoxidase mit Na-Phosphatpuffer 50 mmol/l pH 6,0 verdünnt und für die Tests verwendet.

2. Test:
Zur Durchführung der Tests werden 10 µl Testprobe in einer Dimethylsulfoxid-haltigen Lösung (max. V/V 30%) mit 30 µl der o.g. hergestellten Myeloperoxidaselösung = 1 - 2,5 µg Protein in eine Microtiterplatte (Fa. Greiner) mit glattem Boden gegeben. Die Reaktion wird durch Zugabe eines Testgemisches, bestehend aus 250 µl Na-Phosphatpuffer 50 mmol/l pH 6,0, 10 µl $H_2O_2$ 0,5 x $10^{-3}$ mol/l und 10 µl ortho-Dianisidin 2,5 x $10^{-2}$ mol/l in Dimethylsulfoxid gestartet. Die Zunahme der Extinktion wird bei 30°C am Elisa-Reader bei 490 nm gemessen. Es werden 6 Messungen im Abstand von 2 min durchgeführt und die Extinktionszunahme pro Minute berechnet. Eine Kontrolle, die anstatt einer Testprobe nur die entsprechende Menge Dimethylsulfoxid enthält, wird als 100%-Wert angesetzt und darauf die Hemmung durch die Probe berechnet. Alle Messungen werden als 3-fach Bestimmung durchgeführt.

Die erfindungsgemäße Verbindung (Ia) Beispiel 1 bewirkt in dem oben genannten Test eine Hemmung der Aktivität der Myeloperoxidase, wie in Tabelle 6 dargestellt:

Tabelle 6

| Konzentration Beispiel 1 µmol/l | %-Hemmung |
|---|---|
| 50 | 60 |
| 10 | 45 |
| 1 | 28 |
| 0,1 | 12 |

3. An der Hamsterbackentasche (Präparation nach Duling, MVR 5, 423 - 429, 1973) wurde die Leukozytenadhäsion in kleinen Venolen untersucht. Eine gesteigerte Adhäsion ist bedingt durch die präparative Vorbereitung der Backentasche. Die Substanz vermindert ab 0,1 mg/kg i.v. die Leukozytenadhasion signifikant. In weiteren Versuchen wurden Backentaschen mit $LTB_4$ für 10 min superfundiert. Die Bestimmung der Myeloperoxidase-Aktivität als direktes Maß für die Akkumulation von neutrophilen Leukozyten ergibt Anstiege um 80 - 100% gegenüber der Kontrolle. Mit 1 mg/kg i.v. verhindert der neue Naturstoff (Ia / Beispiel 1) den Anstieg der MPO-Aktivität.

4. In vivo Wirkung von Beispiel 1 (allg. Formel Ia)
Reduktion der Infarktgröße im Ratteninfarktmodell

Methode:
Zur Bestimmung der in vivo Wirkung der Verbindung aus Beispiel 1 narkotisierten (Trapanal i.p.) Wistar Ratten ein Herzinfarkt durch Ligation der linken Koronararterie experimentell induziert. Nach einer 5-minütigen postoperativen Erholungsphase wurde die Verbindung aus Beispiel 1 in einer Dosis von 1 mg/kg gelöst in 100 % DMSO bzw. Lösungsmittel über 6 min in die katheterisierte Vena jugularis der beatmeten Ratten infundiert und die Koranararterie für 30 min okkludiert. Zum Zeitpunkt Minute 25 der Okklusion erfolgt die zweite Applikation der Verbindung aus Beispiel 1 bzw. Lösungsmittel unter den oben beschriebenen Bedingungen. Nach Ende der Infusion und Okklusionsphase wurde die Ligatur geöffnet und das Herz für 90 min reperfundiert.

Bestimmung der Infarktgrösse:
Zur histologischen Färbung des infarzierten Herzgewebes wurde das Herz ex vivo retrograd mit Triphenyltetrazolium Chlorid (TTC. 1,5 %) in 20 mM Kaliumphosphat Puffer für 15 min und mit 0,9 % NaCl für 10 min bei 37°C und mit einem Perfusionsdruck von 100 mm Hg perfundiert Die Infarktgrössen wurden in Gewichtsprozent von infarziertem zu nicht infarziertem Gewebe berechnet und als Mittelwerte und deren Standardabweichungen von 11 mit der Verbindung aus Beispiel 1 behandelten und von 11 mit Lösungsmittel behandelten Ratten in Prozent angegeben.

Ergebnis:
Die Applikation von je 1 mg/kg der Verbindung aus Beispiel 1 vor Okklusion und vor Reperfusion reduzierte die Infarktgrösse von 19,0 ± 6,0 % und 12,2 ± 4,8 %.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formeln (I)/(Ia) enthalten, oder die aus einem oder mehreren Wirkstoffen der Formeln (I)/(Ia) bestehen, sowie Verfahren zur Herstellung dieser Zubereitungen.

Die Wirkstoffe der Formeln (I)/(Ia) sollen in diesen Zubereitungen in einer Konzentration von 0,1 bis 99,5 Gew.-%, bevorzugt von 0,5 bis 95 Gew.-%, der Gesamtmischung vorhanden sein.

Neben den Wirkstoffen der Formeln (I)/(Ia) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formeln (I)/(Ia) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

**Beispiel 1**

**Cyclamenol** (Ia)

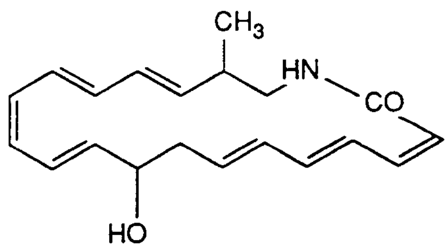

(Ia)

Beispiel I

Herstellung des Impfgutes für die Vorkultur

150 ml sterile, in 1 l-Erlenmeyerkolben befindliche Näturlösung der folgenden Zusammensetzung:

| | |
|---|---|
| Glucose | 4 g |
| Malzextrakt | 10 g |
| Hefeextrakt | 4 g |
| ad 1000 ml Leitungswasser pH 7,3 | |

werden mit Myzelium des Streptomyceten-Stammes MHW 846 beimpft und drei Tage auf der Rundschüttelmaschine mit 240 Upm bei +28°C fermentiert. Die gewachsenen Kulturen dienen als Impfgut für weitere Fermentationsansätze.

Beispiel II

Herstellung der Vorkultur für die Tankfermentation

20 l Nährlösung der unter Beispiel I beschriebenen Zusammensetzung und 20 ml Entschäumer (SAG 5693, Union Carbide) werden in einem 30 l-Kessel mit Rührwerk, Belüftungs- und Temperierungseinrichtung abgefüllt und bei +121°C 50 min sterilisiert. Nach Abkühlen der Nährlösung auf +28°C wird mit 300 ml der wie unter Beispiel I beschriebenen gewonnenen Schüttelkulturen des Streptomyceten-Stammes MHW 846 beimpft, mit 10 l steriler Luft pro min (0,5 VVm) bei 300 Umdrehungen des Rührwerks (Blattrührer) pro Minute belüftet und bei einer Temperatur von +28°C und einem Überlagerungsdruck von 0,5 bar fermentiert. Nach zweitägiger Fermentation wird der Inhalt als Impfgut für einen Ansatz in einem 600 l-Kessel verwendet.

Beispiel III

Fermentation im 600 l-Kessel

400 l Nahrlösung der folgenden Zusammensetzung:

| | |
|---|---|
| Glucose | 5 g |
| Dextrin | 40 g |
| Hefeautolysat | 1,5 g |
| Milchpulver | 10 g |
| ad 1000 ml Leitungswasser | |

werden in einem 600 l-Kessel auf pH 7,0 eingesetllt, 60 min bei +121°C sterilisiert, auf +28°C abgekühlt und mit 20 l des gemäß Beispiel II erhaltenen Impfgutes beimpft. Die Fermentation von MHW 846 wird bei +28°C, einer Belüftung von 200 l steriler Luft pro min (0,5 VVm), bei einem Überlagerungsdruck von 1 bar mit 150 Umdrehungen (Blattrührer) pro Minute durchgeführt. Wenn nach drei- bis sechstägiger Fermentation die erfindungsgemäße Verbindung (1) gebildet worden ist, wird die Kultur geerntet.
Produktbildung: 1 - 2 mg/l.

Beispiel IV

Die gemäß Beispiel III erhaltene Kulturbrühe einer 400 l-Fermentation wird bei pH 6,5 - 7,0 mit 200 - 250 l/h in einem Westfalia-Separator separiert.

Das Mycel wird mit dem gleichen Volumen Methanol vesetzt und 10 min gerührt. Danach werden 30 l Essigsäureethylester zugegeben und weitere 60 min gerührt. Zur Phasentrennung werden 10 - 15 l Wasser zugegeben und die organische Phase abgetrennt.
Die organische Phase wird auf ca. 5 l eingeengt und mittels Chromatographie direkt weiter aufgereinigt.
Nach Abtrennen des Mycels werden 400 l Überstand mit 25 kg Kochsalz versetzt und nacheinander mit 130 l und 30 l Essigsäureethylester extrahiert. Die vereinigten Extrakte werden unter reduziertem Druck auf ca. 5 l eingeengt.

Beispiel V

Weitere Anreicherung durch Mitteldruckchromatographie:

| | |
|---|---|
| Säule | Glassäule 10 x 18 cm |
| Fließrate: | 20 ml/min |
| Detektion: | 280 nm |
| stat.Phase: | Kieselgel Si 60, 70 -230 mesh |
| Laufmittel: | Essigsäureethylester p.a. |

Der Rohextrakt aus Beispiel IV wird weiter aufkonzentriert auf einen Wirkstoffgehalt von ca. 1 mg/kg (nach analyt. HPLC).
Das Säuleneluat wird fraktioniert aufgefangen und einer analytischen Hochdruckflüssigkeitschromatographie gemäß folgenden Parametern unterworfen:

| | |
|---|---|
| Fließmittel: | 45% wäßriges Methanol LiChroSolv |
| Fließrate: | 1 ml/min |
| Detektion: | 285 nm und 220 nm |
| Stat.Phase: | Chrompack 3 x 10 ODS 2 |

Fraktionen, die den Wirkstoff enthalten, werden vereinigt und der Wirkstoff als ca. 60 - 80%iges Präparat aus konzentrierter Lösung mit Petrolether ausgefällt und im Vakuum getrocknet.

Beispiel VI

Isolierung der Reinsubstanz:

Aus dem angereicherten Produkt aus Beispiel IV wird mittels HPLC die Reinsubstanz isoliert.

| | |
|---|---|
| Säule: | Latek 5 x 25 cm ODS 2 5µ |

Fließmittel: 55% wäßriges Methanol
Fließrate: 30 ml/min
Detektion: 285 nm

**Beispiele 2, 3 und 4**

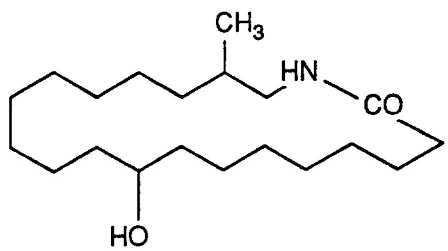

(2)

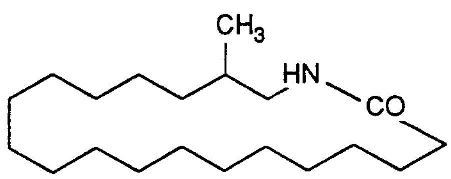

(3)

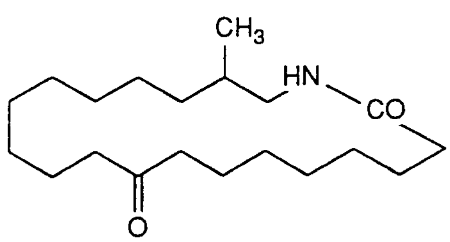

(4)

45 mg der Verbindung aus Beispiel 1 werden in 10 ml Methanol bei 0°C gelöst und unter Rühren nach Zugabe von einer Spatelspitze 10%igem Palladium auf Aktivkohle mit Wasserstoff hydriert. Nach 20 min wird abgebrochen, filtriert, eingedampft und durch Chromatographie an Kiesel 60 gereinigt Man erhält 20 mg nach HPLC und $^{1}H$-NMR einheitliches Produkt der Verbindung des Beispiel 2; $R_f$ (Kieselgel, $CHCl_3$/Methanol 9:1) 0,47, grasgrüne Farbe mit TDM-Reagens (wird violett), UV-inaktiv, farblose Kristalle, Schmp. 84°C.
Das $^{13}C$-NMR-Spektrum der Verbindung aus Beispiel 2 zeigt die vollständige Hydrierung des Doppelbindungssystems.
Das hochaufgelöste EI-MS zeigt den Molpeak bei m/z = 325,2966 (ber. für $C_{20}H_{39}NO_2$: 325,2981).
Die Elementaranalyse hat folgendes Ergebnis:

| | C | H | N |
|---|---|---|---|
| Ber. (%) | 73,8 | 12,1 | 4,3 |
| Gef.:(%) | 73,9 | 10,7 | 4,3 |

Außerdem zeigt die Abb. 6 in der Anlage das IR- und $^{1}H$-NMR-Spektrum der Verbindung aus Beispiel 2.

Neben der Verbindung aus Beispiel 2 entstehen noch geringe Mengen des Deoxygenierungsproduktes der Verbindung aus Beispiel (3) und des Ketons der Verbindung aus Beispiel (4).

Beispiel 5

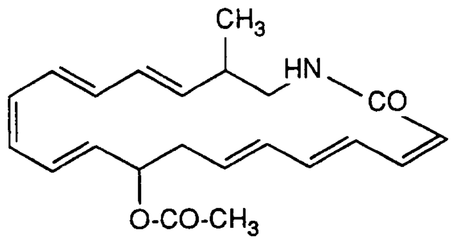

90 mg der Verbindung aus Beispiel 1 werden unter Argon in ca. 2 ml Pyridin gelöst. Man gibt 2 Kristalle 4-(N,N-Dimethylamino)-pyridin (DMAP) und 40 Tropfen Acetanhydrid hinzu und rührt eine Stunde bei RT. Man gibt 220 ml Wasser hinzu und extrahiert 5 x mit Essigester. Die organische Phase wird mit Natriumsulfat getrocknet, eingeengt und durch Chromatographie an Kieselgel gereinigt. Man erhält 40 mg nach HPLC und $^{1}$H-NMR einheitliches Produkt Cyclamenol-Monoacetat, $R_F$ (Kieselgel, Chloroform / Methanol 9:1) 0,84; grüne Farbe mit TDM-Reagens, UV-aktiv (254 nm), farblose Kristalle (Nadeln), Schmp. 260°C, FT-IR-Spektrum (KBr):

Tabelle 8

| IR-Spektrum | |
|---|---|
| Wellenzahl in $cm^{-1}$ | Wellenzahl in cm-$^{1}$ |
| 3380 (sh) | 1535 (m) |
| 3295 (m) | 1445 (w) |
| 3005 (w) | 1365 (m) |
| 2950 (w) | 1240 (vs) |
| 2910 (w) | 1095 (w) |
| 2875 (w) | 1025 (m) |
| 1745 (vs) | 995 (vs) |
| 1650 (s) | 895 (w) |
| 1610 (m) | |

Beispiel 6

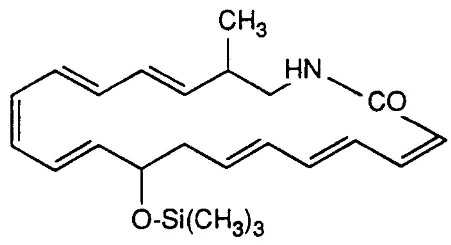

140 mg der Verbindung aus Beispiel 1 werden in 5 ml abs. Acetonitril gelöst. Man gibt bei RT unter Rühren (Argon) eine Lösung von 500 mg N,O-Bis(trimethylsilyl)acetamid in wenig Acetonitril, 0,3 ml trockenes Pyridin und 10 Tropfen Trimethylchlorsilan hinzu. Nach 30 min wird bei RT eingedampft und bei 0,1 Torr getrocknet. Man reinigt durch präp. Dünnschichtchromatographie an Kieselgel mit Dichlormethan / Methanol 9:1 und erhält 40 mg farblosen, amorphen Feststoff Cyclamenol-(Mono)trimethylsilylether, $R_f$ (Kieselgel, $CHCl_3$ / Methanol 9:1) 0,81, grüne Farbe mit TDM-Reagens, UV-aktiv, FT-IR (KBr):

Tabelle 9

| IR-Spektrum | |
|---|---|
| Wellenzahl in $cm^{-1}$ | Wellenzahl in $cm^{-1}$ |
| 3395 (breit, vs) | 1390 (vs) |
| 2955 (m) | 1250 (s) |
| 2920 (sh) | 1100 (breit m) |
| 1720 (sh) | 1015 (m) |
| 1670 (breit, vs) | 890 (w) |
| 1550 (w) | 850 (s) |
| 1475 (w) | 775 (w) |

## Patentansprüche

1. Verbindungen der allgemeinen Formel (I)

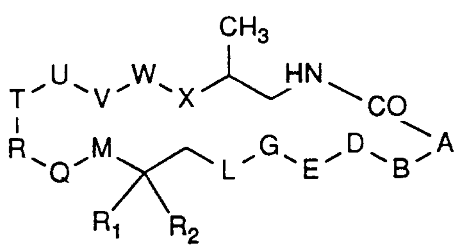

(I)

in welcher

A, B, D, E, G, L, M, Q, R, T, U, V, W und X jeweils für die >$CH_2$-Gruppe stehen
und

$R^1$ und $R^2$ für Wasserstoff stehen
oder

$R^1$ für Wasserstoff steht
und

$R^2$ für Hydroxy steht,
oder

$R^1$ und $R^2$ gemeinsam die =O-Gruppe bilden,
oder

A und B, D und E, G und L, M und Q, R und T, U und V und W und X jeweils für die -CH=CH-Gruppe stehen,

$R^1$ für Wasserstoff steht
und

$R^2$ für Hydroxy steht oder
für die Gruppe der Formel $-O-CO-CH_3$ oder $-O-Si(CH_3)_3$ steht.

2. Arzneimittel enthaltend eine oder mehrere Verbindungen aus Anspruch 1.

3. Arzneimittel nach Anspruch 2 zur Behandlung von akuten oder chronischen Entzündungen.

4. Verwendung der Verbindungen aus Anspruch 1 zur Herstellung von Arzneimitteln.

5. Verfahren zur Herstellung der Verbindungen nach Anspruch 1, dadurch gekennzeichnet, daß man Streptomyces spec. MHW 846 oder eine seiner Mutanten oder Varianten kultiviert, das Cyclamenol aus der Kultur gewinnt und gegebenenfalls derivatisiert.

## Claims

1. Compounds of the general formula (I)

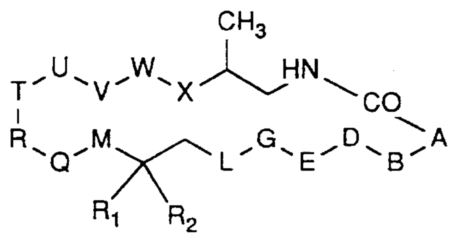

(I)

in which

A, B, D, E, G, L, M, Q, R, T, U, V, W and X in each case represent the $>CH_2$ group
and

$R^1$ and $R^2$ represent hydrogen
or

$R^1$ represents hydrogen
and

$R^2$ represents hydroxyl,
or

$R^1$ and $R^2$ together form the =O group,
or

A and B, D and E, G and L, M and Q, R and T, U and V and W and X in each case represent the -CH=CH-group,

$R^1$ represents hydrogen
and

$R^2$ represents hydroxyl or
represents the group of the formula $-O-CO-CH_3$ or $-O-Si(CH_3)_3$.

2. Medicament containing one or more compounds from Claim 1.

**3.** Medicament according to Claim 2 for the treatment of acute or chronic inflammations.

**4.** Use of the compounds from Claim 1 for the production of medicaments.

**5.** Process for the preparation of the compounds according to Claim 1, characterized in that Streptomyces spec. MHW 846 or one of its mutants or variants is cultured, and the cyclamenol is recovered from the culture and optionally derivatized.

**Revendications**

**1.** Composés de formule générale (I)

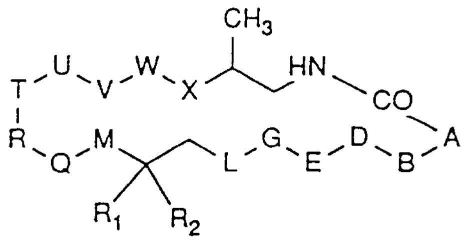

(I)

dans laquelle

A, B, D, E, G, L, M, Q, R, T, U, V, W et X représentent chacun le groupe
$>CH_2-$
et
$R^1$ et $R^2$ représentent de l'hydrogène,
ou bien
$R^1$ est de l'hydrogène
et
$R^2$ est un groupe hydroxy,
ou bien
$R^1$ et $R^2$ forment ensemble le groupe =O,
ou bien
A et B, D et E, G et L, M et Q, R et T, U et V, et W et X représentent dans chaque cas le groupe -CH=CH-,
$R^1$ représente de l'hydrogène
et
$R^2$ est un groupe hydroxy, ou bien
le groupe de formule $-O-CO-CH_3$ ou $-O-Si(CH_3)_3$.

**2.** Médicament contenant un ou plusieurs composés suivant la revendication 1.

**3.** Médicament suivant la revendication 2, destiné au traitement d'inflammations aiguës ou chroniques.

**4.** Utilisation des composés suivant la revendication 1 pour la préparation de médicaments.

**5.** Procédé de production des composés suivant la revendication 1, caractérisé en ce qu'on cultive l'organisme Streptomyces spec. MHW 846 ou l'un de ses mutants ou variants, on isole le cyclaménol de la culture et on le transforme éventuellement en dérivés.

Fig.1

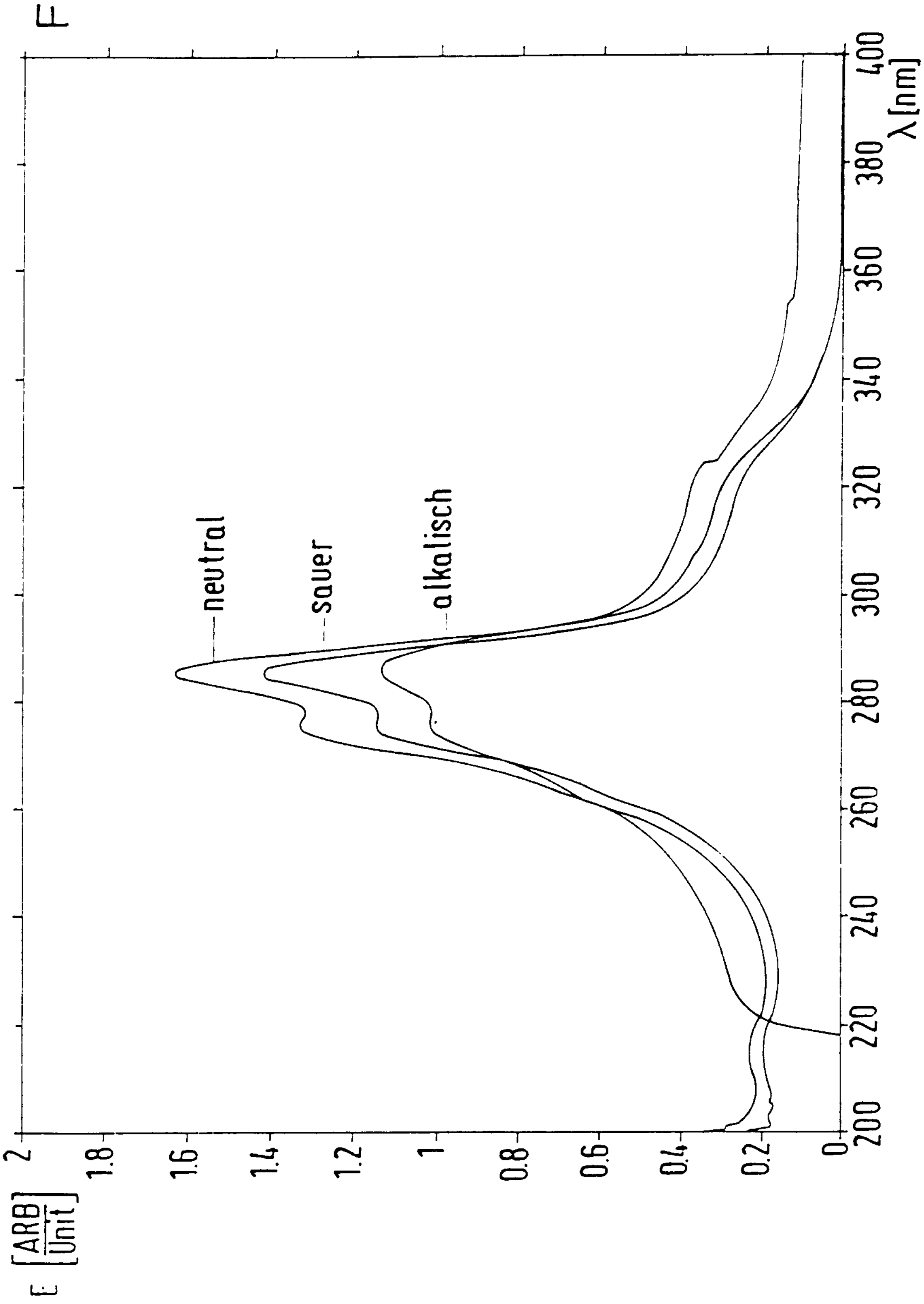
E [ARB/Unit]
2
1.8
1.6
1.4
1.2
1
0.8
0.6
0.4
0.2
0
200
220
240
260
280
300
320
340
360
380
400
λ [nm]
neutral
sauer
alkalisch

Fig.2

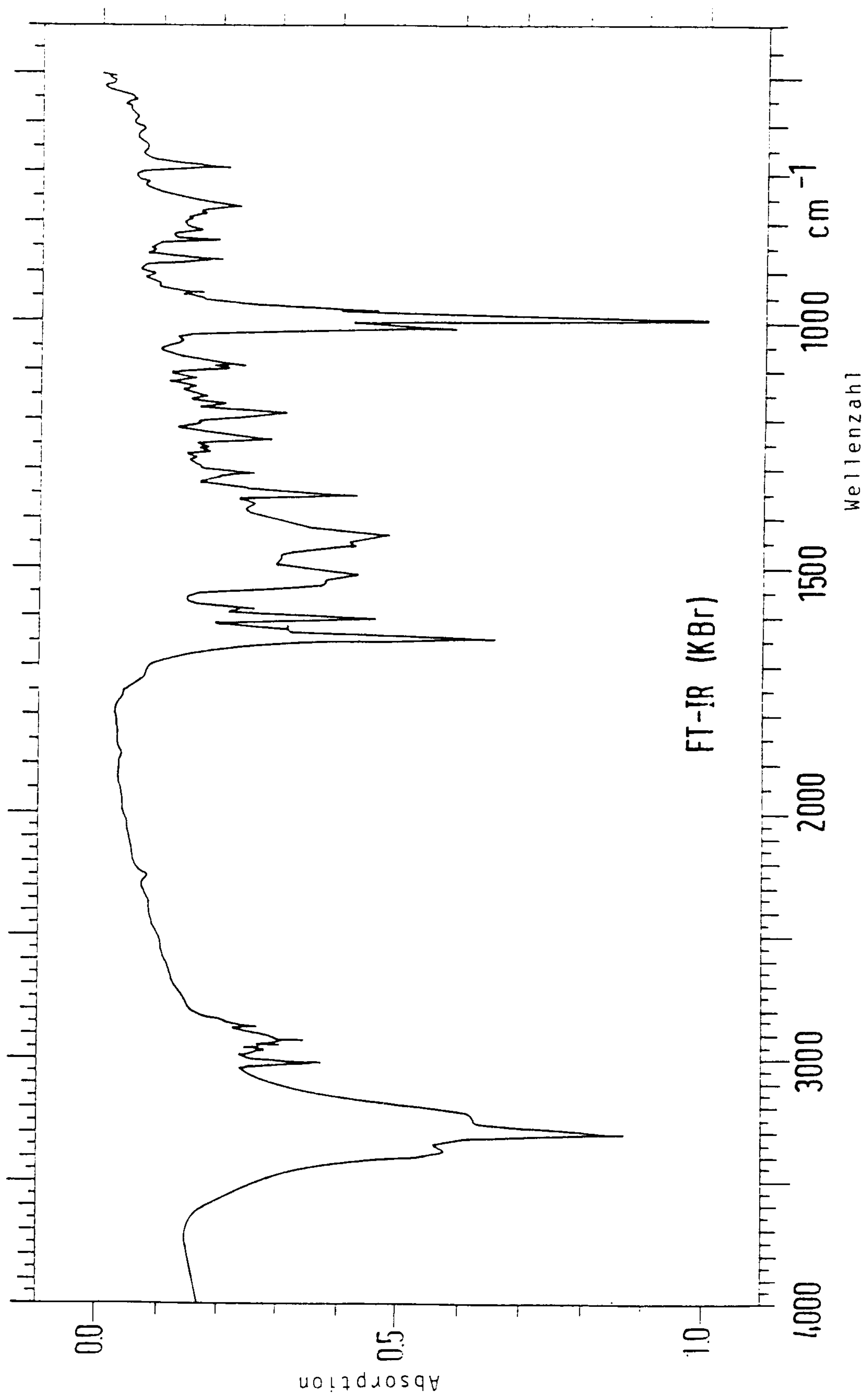
FT-IR (KBr)
Wellenzahl
cm-1
4000
3000
2000
1500
1000
0.0
0.5
1.0
Absorption

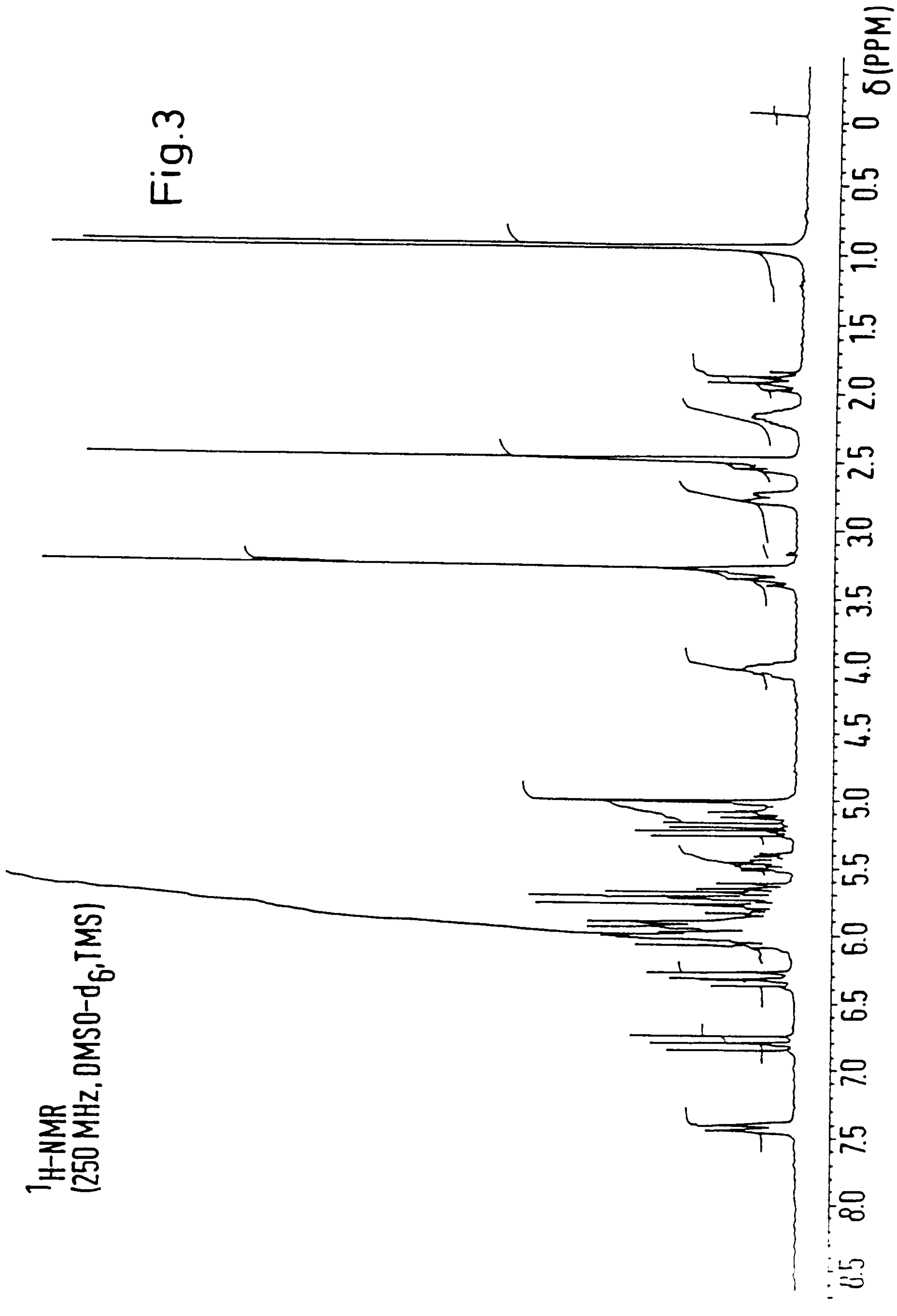

$^1H$-NMR
(250 MHz, DMSO-$d_6$, TMS)
δ (PPM)
0
0.5
1.0
1.5
2.0
2.5
3.0
3.5
4.0
4.5
5.0
5.5
6.0
6.5
7.0
7.5
8.0
8.5


Fig.3